# EUROPEAN PATENT SPECIFICATION

(11) **EP 1 054 698 B1**
(45) Date of publication and mention of the grant of the patent: **22.10.2003**
(21) Application number: 99904950.5
(22) Date of filing: 11.02.1999
(51) Int. Cl.: A61L 11/00

(54) **A METHOD AND APPARATUS FOR TREATING CLINICAL WASTE**
VORRICHTUNG UND VERFAHREN ZUR AUFBEREITUNG VON KLINISCHEN ABFÄLLEN
PROCEDE ET APPAREIL POUR LE TRAITEMENT DE DECHETS HOSPITALIERS

(30) Priority: 11.02.1998 GB 9802906
(43) Date of publication of application: 29.11.2000
(73) Proprietor: Eurocare Environmental Services Limited, Newcastle upon Tyne NE6 4LT (GB)
(72) Inventor: TARN, David Charles, High Shincliffe, Durham City DH1 2PQ (GB)
(74) Representative: Dealtry, Brian
(86) International application number: GB9900271
(87) International publication number: WO99040951

(56) References cited:
- EP-A- 0 277 507
- WO-A-93/06418
- WO-A-95/18679
- CA-A- 1 316 324
- DE-C- 3 705 364
- US-A- 5 570 845
- US-A- 5 656 248

## Description

The present invention relates to a method and apparatus for treating clinical waste.

In particular the invention relates to a method and apparatus for disinfecting clinical waste by heat treatment such that it may be disposed safely in land fills.

Systems for such treatment of clinical waste are known, for example see US 5277136. In US patent 5277136 there is disclosed apparatus which includes a heated disinfecting chamber in which there is located several side by side hollow flighted screw conveyors. The screw conveyors are rotated to transport waste material through the disinfecting chamber and are internally heated with heated fluid so as to indirectly heat the waste material. The waste material is heated to a sufficient temperature and maintained in contact with the screw conveyors for a sufficient period of time in order to ensure disinfection of the waste material. The period of time to which the waste material is exposed to heat is controlled by a computer control system which controls the rate of rotation of the screw conveyors and so varies the resident time of the waste material within the disinfecting chamber.

The waste material which is to be processed widely varies in its constitution and volume and so the computer control system disclosed in US 5277136 has to continually vary the rate of rotation of the screw conveyors to maintain the desired heat exposure time within the disinfecting chamber. Accordingly the throughput of the system of US 5277136 continuously changes and this is a drawback since it complicates processing of the waste material and complicates the processing control of the apparatus.

An aim of the present invention is to provide an improved heat disinfection method and apparatus.

According to one aspect of the present invention there is provided apparatus for the heat treatment of clinical waste, the apparatus including an enclosed disinfecting chamber having a waste inlet and a waste outlet, a valve located at the inlet to provide a gas seal whilst permitting waste to pass into the chamber, conveyor means for conveying the waste in a conveying direction from the inlet to the outlet, the conveyor means being driven at a desired constant speed, heating means for heating the conveyor means and/or chamber housing so as to heat the interior of the chamber for indirect heating of the waste whilst it is being conveyed between the inlet and outlet, temperature sensing means for sensing the temperature of the interior of the chamber and computer control means operatively connected to the temperature sensing means and the heating means, the computer control means controlling the heating means so as to maintain a desired steady state temperature profile along the conveying direction within the interior of the chamber between said inlet and outlet, the temperature profile along a desired length of the chamber interior being above a desired minimum disinfecting temperature and having a maximum temperature adjacent to said inlet.

Preferably, the apparatus further includes water dispensing means for dispensing water onto the waste in the region of the inlet, and relative humidity sensing means for sensing the relative humidity within the interior of the chamber, the water dispensing means and the relative humidity sensing means being operatively connected to said computer control means, the computer control means being arranged to control the amount of water dispensed by the water dispensing means so as to maintain the relative humidity within the chamber above a predetermined minimum value.

Preferably the apparatus further includes gas exhaust means communicating with the interior of the chamber, an aspirator communicating with the exhaust means via filter means, the aspirator being capable of drawing gases from the chamber at varying rates of flow, and pressure sensing means for sensing pressure within the interior of the chamber, the computer control means being operatively connected to-the aspirator and the pressure sensing means and being arranged to vary the flow of gas drawn by the aspirator in order to maintain a desired pressure within the chamber.

Preferably the filter means communicates with the exhaust means via condensing means, the condensing means being operable to condense water vapour in the exhaust gases prior to reaching the filter means. Preferably the condensed water is fed to the water dispensing means.

Preferably the apparatus includes an enclosed feed conveyor for feeding clinical waste to said waste inlet, the feed conveyor being heated, preferably by condensed water from said condensing means, so as to pre-heat the clinical waste prior to entry into said chamber.

Preferably the apparatus further includes a takeaway conveyor located at the outlet of the chamber, the takeaway conveyor being arranged to receive treated waste from said outlet and transport the treated waste to a discharge location, the takeaway conveyor preferably being heated so as to maintain the temperature of the treated waste above a desired minimum temperature.

Preferably steam injection means are provided in the region of said inlet for effecting partial disinfection and pre-heating of the clinical waste by directly heating the clinical waste prior to being indirectly heated in said chamber.

According to another aspect of the present invention there is provided a method of heat treating clinical waste, the method including conveying waste to be treated through a disinfecting chamber at a substantially constant rate, applying heat so as to indirectly heat the waste as it passes through the chamber, monitoring the temperature within the interior of the chamber and varying the applied heat in response to the monitored temperature in order to maintain a desired steady state temperature profile within the interior of the chamber and along the direction of conveyance of the material, said temperature profile being above a desired minimum disinfecting temperature for a desired length along said chamber and having a maximum temperature adjacent to said inlet.

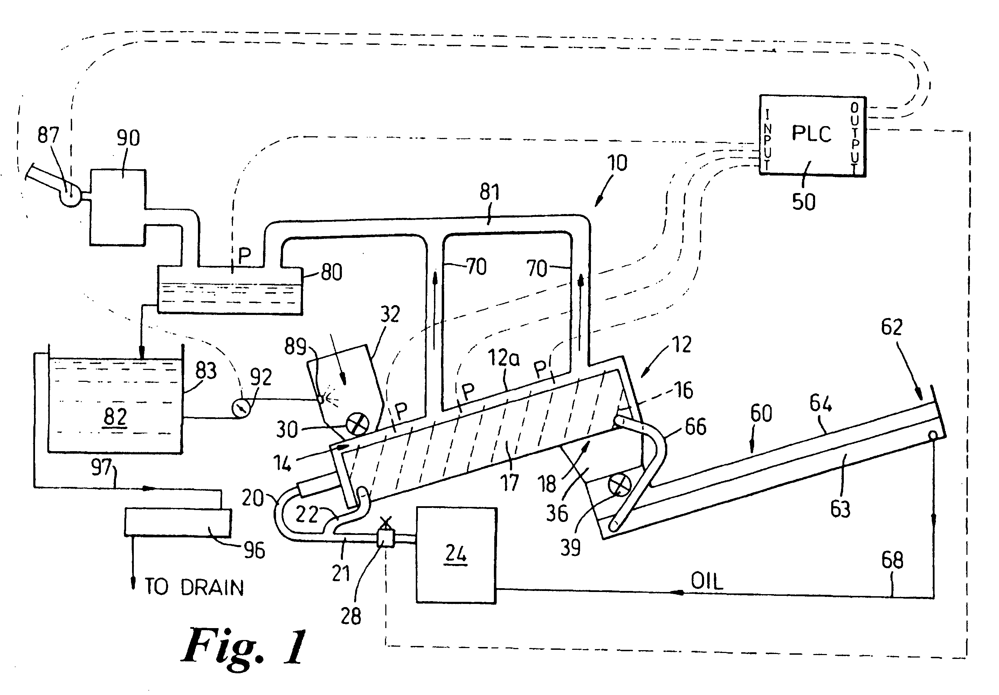

Various aspects of the present invention are hereinafter described with reference to the accompanying drawings, in which:-
Figure 1 is a diagrammatic representation of apparatus according to one embodiment of the present invention;
Figure 2 is a schematic perspective view of the feed hopper and comminuting means;
Figure 3 is a schematic graphical representation showing a desired heating temperature profile for indirectly heating the clinical waste;
Figure 4 is a schematic view of an upper part of the disinfecting chamber shown in Figure 1;
Figure 5 is a diagrammatic representation of a modification to the apparatus shown in Figure 1;
Figures 6 to 8 are schematic views of further modifications to the apparatus of Figure 1.

The heat treatment apparatus according to one embodiment of the present invention is generally shown at 10. The apparatus 10 includes a heat disinfecting chamber 12 having an inlet 14 through which waste to be treated is introduced into the chamber.

Waste material to be treated is fed to the inlet 14 via a hopper 32. A conventional rotary air lock 30 is located between the hopper 32 and the inlet 14 and acts as a gas seal between the interior of the chamber and the hopper 32 whilst permitting waste material to pass therethrough.

The chamber 12 has an enclosed interior in which is housed conveying means 16 for conveying waste material from the inlet to an outlet 18 at which the material is discharged from the chamber 12.

The waste material is discharged from the outlet 18 via an enclosed discharge chute 36 onto a takeaway conveyor 60.

A rotary air lock 39 is located between the chute 36 and the conveyor 60 so as to provide a gas seal between the interior of the chamber 12 and the conveyor 60 whilst permitting treated waste material to pass therethrough.

The conveying means 16 preferably comprises one or more screw conveyors. The screw conveyors are preferably of the hollow type, as for example described in US patent 5277136. More preferably two or more side by side screw conveyors are provided which are arranged to agitate the waste material as it is being conveyed toward the outlet 18. Agitation of the material is preferred since it facilitates dissipation of the applied heat to the waste material

The conveyor means 16 is operated at a substantially constant rate such that the rate of travel of the waste material through the chamber 12 is substantially constant. The rate of travel chosen is dependant upon the volume of waste material which can be conveyed through the chamber 12 and the required resident time of the waste material within the chamber 12. In this respect the rate of travel is preferably chosen to be the rate required for transporting the maximum volume of waste which can be passed through chamber 12 for a minimum resident time for achieving a desired degree of disinfection.

Thus when the conveyor means 16 is fully loaded with the maximum volume of clinical waste, all the waste will be disinfected to a desired degree.

The chamber 12 is preferably provided with a surrounding fluid jacket 17 in order to enable the interior of the chamber to be heated.

Heating of the interior of the chamber 12 is preferably achieved by supplying heated fluid, preferably oil, to the interior of the hollow screw conveyors and/or to the fluid jacket 17. Accordingly a fluid heater 24 is provided. The heater 24 has a main fluid outlet conduit 21 which communicates with branch conduits 20 and 22 which in turn communicate with the interior of the hollow screw conveyors 16 and the fluid jacket 17. A flow control valve 28 is located in the main conduit 21 and is arranged to control flow of heating fluid from the heater 24.

The temperature within the interior of the chamber 12 is monitored, preferably by a series of probes P located in the roof 12a of the chamber.

Since the rate of travel of the waste through chamber 12 is substantially constant, it is possible to achieve a steady state temperature profile within the chamber 12 which varies in a desired predictable manner as a function of the distance travelled by the waste material through the chamber 12 in its passage from inlet 14 to outlet 18.

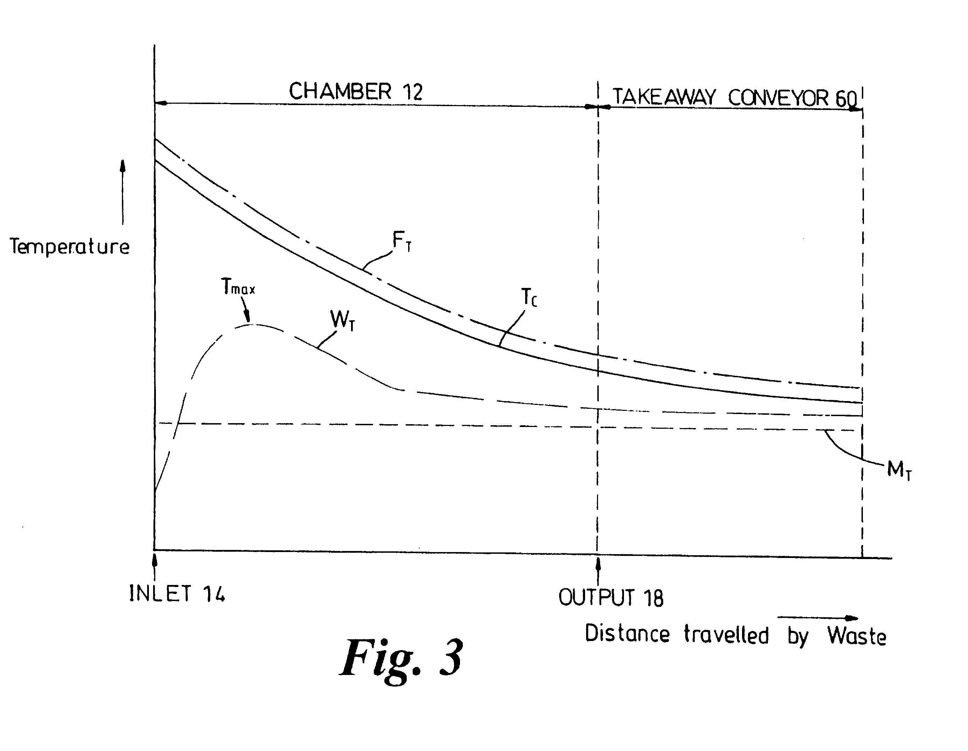

A desired temperature profile is schematically shown in Figure 3 by plot T_{C}. Plot T_{C} shows the temperature of the interior of the chamber 12 as being a maximum adjacent to the inlet 14 and as progressively decreasing toward the outlet 18.

The probes P are connected to a computer control means 50. The control means 50 is connected to the fluid heater 24 and flow control valve 28 and is arranged to control the fluid heater 24 and flow control valve 28 so as to maintain the interior temperature of the chamber 12 in accordance with plot T_{C}.

Since the heat loss along the chamber 12 is substantially constant it is possible to maintain the heat profile of plot T_{C} by varying the temperature and/or flow rate of the heated fluid at the point of entry to the chamber 12. This is shown schematically by plot F_{T} which is a plot of the heated fluid temperature as it passes through chamber 12.

It is known that in order to disinfect clinical waste by heat, it is necessary to expose the clinical waste to a minimum disinfecting temperature of about 104°C (marked as plot M_{T} in Figure 3) for a predetermined minimum period of time.

Accordingly the temperature profile within the chamber 12 (viz. plot T_{C}), is always maintained above the minimum disinfecting temperature for the minimum distance the clinical waste needs to travel in order to be exposed to said minimum disinfecting temperature for said predetermined minimum period of time.

In the present invention, the minimum temperature above which the interior of the chamber 12 is maintained is preferably about 105°C.

The temperature of the clinical waste as it passes through chamber 12 is shown by plot W_{T}. As indicated by plot W_{T}, when the waste first enters chamber 12 it is exposed to a high temperature and so its temperature is quickly raised from ambient temperature to a maximum temperature Tₘₐₓ which is far in excess of 100°C. Accordingly moisture within the clinical waste is quickly converted to steam in the region of the chamber 12 adjacent to inlet 14.

As the waste progresses toward the outlet 18, the moisture content within the waste decreases to such an extent that the treated clinical waste emerging from outlet 18 is substantially completely dry.

The amount of heat applied to the disinfecting chamber 12 is such as to cause moisture in the waste material to be driven off as steam. The generated steam assists in disinfecting the material and agitation of the material as it is being conveyed increases contact between the material and the steam and so optimises the disinfecting effect of the steam. As indicated above, the interior of the chamber is gas sealed at its inlet 14 and outlet 18 and so the generated steam acts to pressurise the interior of the chamber 12. In order to permit the generated steam to escape, one or more exhaust conduits 70 are provided which extend generally upwardly from the roof 12a of the chamber 12. Preferably a plurality of exhaust conduits 70 are provided spaced along the length of the chamber 12.

The conduits 70 preferably communicate with a condenser 80 via a common conduit 81 whereat the exhausted steam is condensed. The condensed water is fed to a storage tank 83 to provide a reservoir of hot water 82. The reservoir 82 communicates with a dispensing jet 89 located within the hopper 32 via a metering pump 92. The metering pump 92 is controlled by the control means 50.

Excess water is allowed to overflow from tank 83 to a heat exchanger 96 via conduit 97 and from the heat exchanger to drain.

Advantageously the conduit 81 is slightly inclined downwardly towards condenser so that condensate from steam condensing within conduit 81 drains off into the condenser 80 instead of returning to the chamber 12. The condenser 80 communicates with an aspirator 87 via a filter 90.

The filter acts to remove harmful gas borne particles and microbes in the exhaust gases before the exhaust gases are discharged to atmosphere.

The aspirator 87 is preferably a variable speed fan, the speed of operation being controlled by control means 50. The control means 50 is preferably arranged to control the speed of the fan in response to pressure sensed within the interior of the chamber 12 such that a desired pressure within the chamber 12 is maintained.

Preferably the pressure within the chamber 12 is maintained so as to be slightly above atmospheric pressure such that the generated steam generates a positive pressure to cause flow of the steam out of the chamber 12. Preferably the pressure within the chamber 12 is about 60 millibars above atmospheric pressure.

It is however envisaged that the pressure within the chamber 12 may be maintained slightly below atmospheric pressure.

The relative humidity within the chamber is sensed and if the sensed relative humidity is below a predetermined minimum addition water is introduced into the waste passing through the hopper by the control means 50 activating the pump 92 to dispense water from the reservoir 82. The dispensed water is hot and requires less heating than if it were cold. The additional water increases the amount of water available for generating steam and so increases the relative humidity.

The control of the relative humidity within the interior of the chamber is desirous since it, enables the temperature of the heating fluid entering the disinfecting chamber to be relatively high for rapidly heating up the waste material to the desired disinfection temperature without risk of the waste material catching fire. The inlet temperature of the oil is preferably in the range of about 140 to 155°C.

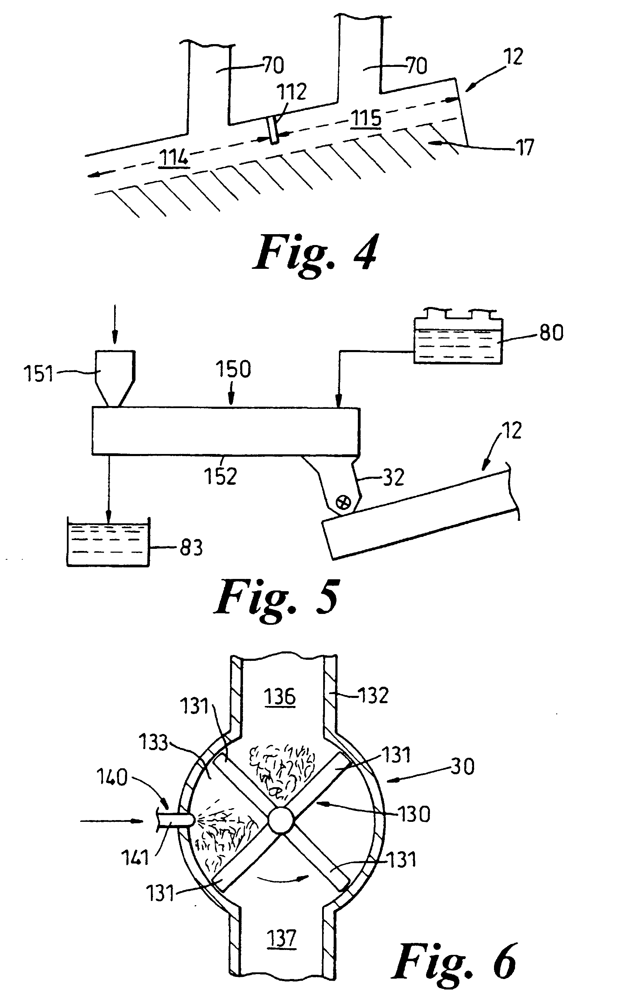

As indicated above the moisture content is adjusted by measuring the relative humidity in the chamber 12. This is preferably achieved by providing a dividing plate 112 (see Figure 4) which in effect acts as a weir to restrict movement of air flow along the chamber 12 in a direction from inlet 14 to outlet 16. This in effect divides the chamber 12 into two distinct relative humidity zones viz. a first zone 114 and a second zone 115.

A probe P is located in zone 114 and a probe P is located in zone 115 in order to independently measure the relative humidity in each zone.

Due to the presence of plate 112, there is a sharply defined difference in relative humidity values between zones 114 and 115 and in view of this it is possible to accurately control the moisture content of the clinical waste.

Accordingly, if the measured relative humidity in zone 114 drops below a predetermined minimum level, say about 90%, controller 50 acts to inject water through jet 89. When the measured relative humidity in zone 115 has risen above a predetermined maximum value, say 10%, controller 50 acts to inhibit injection of water through jet 89.

Preferably the plate 112 is located such that the ratio of the length of the first zone 114 to the second zone 115 is approximately 1:2.

The takeaway conveyor 60 is preferably arranged to heat material as it is conveyed to its discharge end 62. Preferably the conveyor 60 comprises one or more screw conveyors rotatably housed in a casing 64. Preferably heating of the material is achieved by using hollow screw conveyors and/or providing the casing with a fluid jacket 63. Advantageously heated fluid is supplied from the chamber 12, for example via a conduit 66 and is returned to the fluid heater 24 via a conduit 68.

The conveyor 60 is preferably utilised to maintain the temperature of the material deposited thereon above the minimum temperature M_{T}. In this way the clinical waste material continues to be exposed to a disinfecting temperature after emerging from the chamber 12 and so continues to undergo a disinfecting action. This is advantageous in that it enables the resident time of the material in the chamber 12 to be reduced and so permits a greater throughput of material for a given size of chamber 12.

The conveyor 60 may be open to atmosphere since the material deposited from the chamber will have a moisture content below a minimum predetermined value such that harmful vapours will not be emitted.

Preferably the hopper 32 includes comminuting means 130 which comminute the material to a desired maximum particle size, for example about 1 inch cube.

The comminuting means 130 preferably comprises a pair of counter rotating toothed rollers 131 having intermeshing teeth (not shown) which co-operate to comminute the material on rotation of the rollers. Such comminuting means 130 are well known.

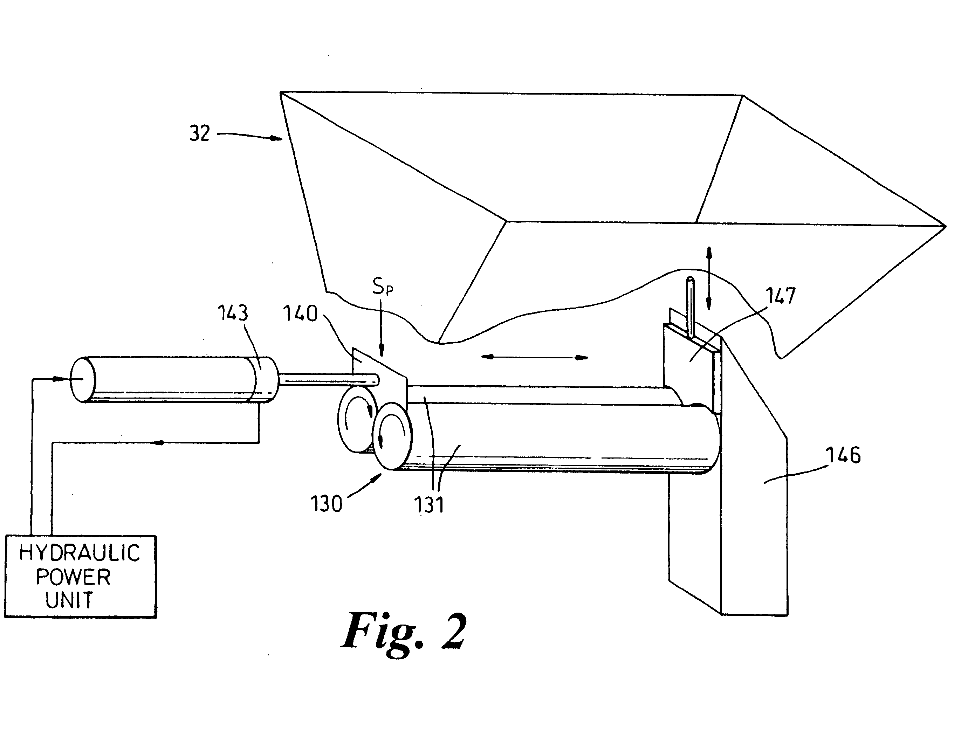

Preferably the rollers are driven by electric motors (not shown). Preferably the hopper houses a retractable tooth scraper blade 140 which in normal use is located in a retracted stowed position S_{P} as seen in Figure 2. The scraper blade 140 is provided to enable the rollers 131 to be cleared of an obstructive object in the event that material is deposited onto the rollers which causes them to stall. This is likely, for instance if a metal object is deposited onto the rollers.

In the event that the rollers are stalled, the stalling of the rollers 131 causes an increase in the electric current being consumed by the electric motors driving the rollers. This increase in current is sensed by the control means 50. The control means 50 operates the motors to reverse the direction of rotation of the rollers for say one revolution and then actuates a ram 143 to advance the scrapper blade 140 longitudinally along the rollers 131. In advancing, the scrapper blade passes over the roller teeth and pushes the obstructing object to a discharge chute 146. The chute 146 has an access door 147 which is normally closed. The door 147 is opened under the control of control means 50 to enable the obstructive material to pass into chute 146.

After the blade has been fully advanced, the control means actuates the ram 143 to return the blade 141 to its stowed position S_{P}. The electric motors are then actuated to rotate in the comminuting direction. If the blockage has not been cleared, the above process of advancing the blade is repeated. If the blockage remains after repeating this process a predetermined number of times, the control means 50 shuts down the apparatus to enable the blockage to be removed manually.

Preferably probes P are each in the form of a combined transducer capable of sensing of the pressure, temperature and relative humidity within the chamber 12.

An optional modification to apparatus 10 is illustrated in Figure 5 wherein a feed conveyor 150 is provided. The feed conveyor 150 is enclosed and has an outlet communicating with hopper 32. Clinical waste is fed into the feed conveyor 150 via a hopper 151.

Preferably the comminuting means 130 is located within hopper 151 instead of hopper 32. The feed conveyor 150 preferably has a hollow casing 152 housing one or more screw conveyors. Preferably hot water from the condensing means 80 is fed via the hollow casing 152 and/or screw conveyors housed in the casing 152 to the tank 83.

Accordingly, the feed conveyor 150 acts to indirectly pre-heat the clinical waste prior to deposit in hopper 32. This has the advantage of raising the temperature of the clinical waste such that by the time it is deposited in hopper 32 it has attained a fairly consistent value irrespective of its temperature when deposited in hopper 152. This helps to avoid excessive cooling of the chamber 12 as the clinical waste enters the chamber 12 and so helps to minimise disturbance of the steady state temperature profile in the region of inlet 14.

In addition, feeding of water from the condensing means 80 to the feed conveyor 150 enables heat to be recovered and fed back into the system.

A further optional modification to apparatus 10 is shown in Figure 6 wherein steam is injected into the clinical waste prior to it being deposited into chamber 12.

In Figure 6, the rotary air lock 30 is shown as including a rotary paddle wheel 130 having four paddles 131. The paddles 131 co-operate with the casing wall 132 to create a succession of sealed chambers 133 which convey the clinical waste from an inlet 136 to an outlet 137 of the air lock 30.

Steam injection means 140, preferably in the form of one or more nozzles 141, are mounted on the casing wall 132 so as to inject steam into a sealed chamber 133 as it moves from the inlet 136 to the outlet 137.

In view of the confined space within each chamber 133, the injected steam tends to penetrate through the clinical waste contained in the chamber 133 and so acts to rapidly heat and partially disinfect the clinical waste by direct heat as it passes from inlet 136 to 137.

Again this has the advantage of pre-heating the clinical waste prior to entry into chamber 12 and so reduces the cooling effect on the steady state temperature within chamber 12.

Preferably the temperature of the steam and the duration of its injection are controlled by the controller 50.

It will be appreciated that injection of steam through injection means 140 also assists in controlling the moisture content of the clinical waste.

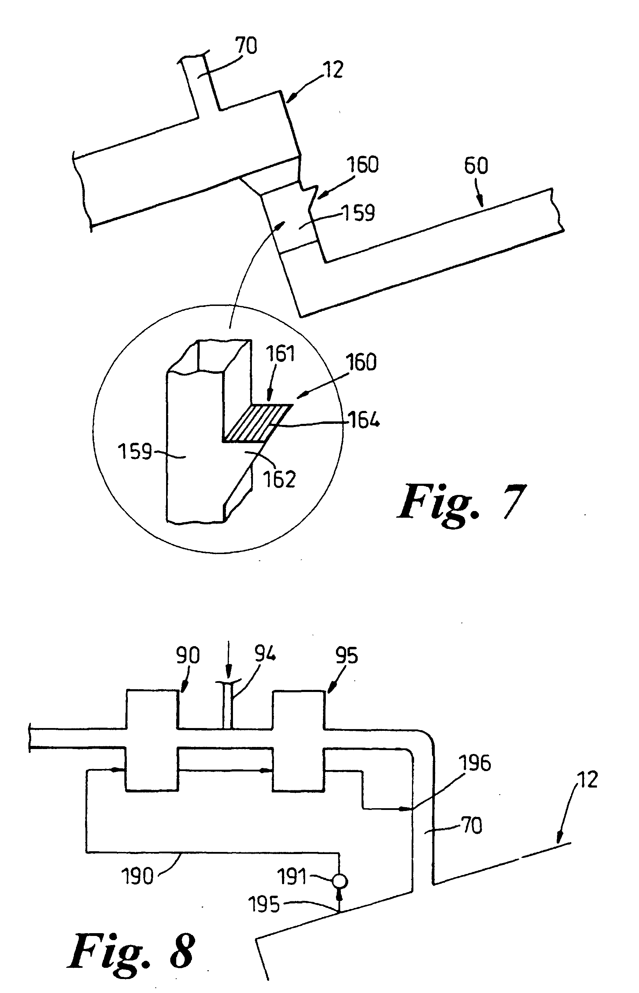

A further optional modification to apparatus 10 is illustrated in Figure 7 wherein the second air lock 39 is omitted such that an unrestricted flow of air is permitted from the interior of conveyor 60 to chamber 12.

In this modification, the aspirator 87 is controlled to provide a slightly negative pressure within chamber 12 such that air is always drawn into chamber 12.

Preferably the interconnecting ducting 159 between chamber 12 and conveyor 60 is provided with venting means 160 which acts to vent the interior of chamber 12 to atmosphere so as to ensure that an air flow is provided into chamber 12 irrespective of the amount of clinical waste travelling into conveyor 60 from chamber 12. Preferably venting means 160 comprises an air inlet 161 which is formed on a branched duct wall 162 so as to be isolated from the flow of clinical waste along the ducting 159. Preferably the air inlet 161 is covered with a grille 164.

A further optional modification to apparatus 10 is illustrated in Figure 8 wherein in addition to the filter 90 there is provided an ammonia scrubber 95 containing carbon eating enzymes. Preferably ammonia eating enzymes are introduced into the conduit inbetween the scrubber 95 and filter 90 via conduit 94. These enzymes permeate the biomass held on the filter 90 and assist in reducing odours. Preferably in order to maintain the filter 90 and scrubber 95 at an optimum temperature, preferably about 21°C, for enzyme activity, hot gases are drawn through a bypass conduit 190 and fed to heat exchangers associated with both filter 90 and scrubber 95. The flow along conduit 190 is controlled by a valve 191 by controller 50. The inlet 195 to conduit 190 is located at a suitable position in chamber 12 in order to draw hot gases from its interior. The conduit 190 communicates via an outlet 196 with one of the conduits 70 to create a negative pressure for drawing gases along conduit 190.

## Claims

1. Apparatus for the heat treatment of clinical waste, the apparatus including an enclosed disinfecting chamber (12) having a waste inlet (14) and a waste outlet (18), a valves (30) located at the inlet to provide a gas seal whilst permitting waste to pass into the chamber, conveyor means (16) for conveying the waste in a conveying direction from the inlet to the outlet, the conveyor means being driven at a desired constant speed, heating means (17, 24) for heating the conveyor means and/or chamber housing so as to heat the interior of the chamber for indirect heating of the waste whilst it is being conveyed between the inlet and outlet, temperature sensing means for sensing the temperature of the interior of the chamber and computer control means (50) operatively connected to the temperature sensing means and the heating means, the computer control means controlling the heating means so as to maintain a desired steady state temperature profile along the conveying direction within the interior of the chamber between said inlet and outlet, the temperature profile along a desired length of the chamber interior being above a desired minimum disinfecting temperature and having a maximum temperature adjacent to said inlet.

2. Apparatus according to Claim 1 further including water dispensing means (89, 92) for dispensing water onto the waste in the region of the inlet, and relative humidity sensing means for sensing the relative humidity within the interior of the chamber, the water dispensing means and the relative humidity sensing means being operatively connected to said computer control means (50), the computer control means being arranged to control the amount of water dispensed by the water dispensing means so as to maintain the relative humidity within the chamber above a predetermined minimum value.

3. Apparatus according to Claim 1 or 2 further including gas exhaust means communicating with the interior of the chamber, an aspirator communicating with the exhaust means via filter means, the aspirator being capable of drawing gases from the chamber at varying rates of flow, and pressure sensing means for sensing pressure within the interior of the chamber, the computer control means being operatively connected to the aspirator and the pressure sensing means and being arranged to vary the flow of gas drawn by the aspirator in order to maintain a desired pressure within the chamber.

4. Apparatus according to Claim 3 wherein the filter means communicates with the exhaust means via condensing means, the condensing means being operable to condense water vapour in the exhaust gases prior to reaching the filter means.

5. Apparatus according to Claim 4 including an enclosed feed conveyor for feeding clinical waste to said waste inlet, the feed conveyor being heated, preferably by condensed water from said condensing means, so as to pre-heat the clinical waste prior to entry into said chamber.

6. Apparatus according to any preceding claim further including a takeaway conveyor located at the outlet of the chamber, the takeaway conveyor being arranged to receive treated waste from said outlet and transport the treated waste to a discharge location, the takeaway conveyor preferably being heated so as to maintain the temperature of the treated waste above a desired minimum temperature.

7. Apparatus according to any preceding claim wherein steam injection means are provided in the region of said waste inlet for effecting partial disinfection and pre-heating of the clinical waste by directly heating the clinical waste prior to being indirectly heated in said chamber.

8. A method of heat treating clinical waste, the method including conveying waste to be treated through a disinfecting chamber at a substantially constant rate, applying heat so as to indirectly heat the waste as it passes through the chamber, monitoring the temperature within the interior of the chamber and varying the applied heat in response to the monitored temperature in order to maintain a desired steady state temperature profile within the interior of the chamber and along the direction of conveyance of the material, said temperature profile being above a desired minimum disinfecting temperature and having a maximum temperature adjacent to said inlet.

## Patentansprüche

1. Vorrichtung für die Hitzebehandlung von Klinikabfall, wobei die Vorrichtung eine umschlossene Desinfektionskammer (12) umfasst, die folgende Bestandteile aufweist: einen Abfalleinlaß (14) und einen Abfallauslaß (18), ein Ventil (30), das am Einlaß angeordnet ist, um eine Gasdichtung zu schaffen und es dabei dem Abfall zu ermöglichen, in die Kammer zu gelangen, Fördereinrichtungen (16) zum Fördern des Abfalls in einer Förderrichtung vom Einlaß zum Auslaß, wobei die Fördereinrichtungen mit einer gewünschten konstanten Geschwindigkeit angetrieben werden, Heizeinrichtungen (17, 24) zum Heizen der Fördereinrichtungen und/oder des Kammergehäuses, um so das Innere der Kammer für eine indirekte Erhitzung des Abfalls zu heizen, während er zwischen dem Einlaß und dem Auslaß weitertransportiert wird, Temperaturmeßeinrichtungen zum Messen der Temperatur des Inneren der Kammer und Computer-Steuereinrichtungen (50), die arbeitsmäßig mit den Temperaturmeßeinrichtungen und den Heizeinrichtungen verbunden sind, wobei die Computer-Steuereinrichtungen die Heizeinrichtungen so steuern, daß ein gewünschtes stationäres Temperaturprofil längs der Förderrichtung im Inneren der Kammer zwischen dem Einlaß und dem Auslaß aufrechterhalten wird, wobei das Temperaturprofil längs eines gewünschten Abschnitts des Kammerinneren oberhalb einer gewünschten minimalen Desinfektionstemperatur liegt und in der Nähe des Einlasses eine Maximaltemperatur aufweist.

2. Vorrichtung nach Anspruch 1, die weiterhin Wasserabgabeeinrichtungen (89, 92) umfasst, um Wasser auf den Abfall im Bereich des Einlasses abzugeben, sowie Meßeinrichtungen für die relative Feuchtigkeit, um die relative Feuchtigkeit im Inneren der Kammer zu messen, wobei die Wasserabgabeeinrichtungen und die Meßeinrichtungen für die relative Feuchtigkeit arbeitsmäßig mit den Computer-Steuereinrichtungen (50) verbunden und die Computer-Steuereinrichtungen so ausgebildet sind, daß sie die Menge des Wassers steuern, die von den Wasserabgabeeinrichtungen abgegeben wird, um so die relative Feuchtigkeit in der Kammer oberhalb eines vorbestimmten Minimalwertes zu halten.

3. Vorrichtung nach Anspruch 1 oder 2, die weiterhin Gasabgabeeinrichtungen umfasst, die mit dem Inneren der Kammer in Verbindung stehen, eine Ansaugvorrichtung, die mit den Abgaseinrichtungen über Filtereinrichtungen in Verbindung steht, wobei die Ansaugvorrichtung in der Lage ist, Gase aus der Kammer mit sich ändernden Strömungsraten abzuziehen, sowie Druckmeßeinrichtungen, die dazu dienen, den Druck im Inneren der Kammer zu messen, wobei die Computer-Steuereinrichtungen arbeitsmäßig mit der Ansaugvorrichtung und den Druckmeßeinrichtungen verbunden und so ausgebildet sind, daß sie den Gasstrom variieren, der von der Ansaugvorrichtung abgezogen wird, um in der Kammer einen gewünschten Druck aufrechtzuerhalten.

4. Vorrichtung nach Anspruch 3, bei der die Filtereinrichtungen mit den Abgaseinrichtungen über Kondensiereinrichtungen in Verbindung stehen, wobei die Kondensiereinrichtungen betreibbar sind, um Wasserdampf in den Abgasen zu kondensieren, bevor diese die Filtereinrichtungen erreichen.

5. Vorrichtung nach Anspruch 4, die einen umschlossenen Zuführförderer umfasst, um Klinikabfall zum Abfalleinlaß zu fördern, wobei der Zuführförderer vorzugsweise durch kondensiertes Wasser von den Kondensiereinrichtungen erhitzt wird, um so den Klinikabfall vor dem Eintritt in die Kammer vorzuheizen.

6. Vorrichtung nach einem der vorhergehenden Ansprüche, die weiterhin einen Abführförderer umfasst, der am Auslaß der Kammer angeordnet ist, wobei der Abführförderer so angeordnet ist, daß er behandelten Abfall vom Auslaß erhält und den behandelten Abfall zu einer Abgabestelle transportiert, wobei der Abführförderer vorzugsweise so geheizt wird, daß die Temperatur des behandelten Abfalls über einer gewünschten Minimaltemperatur gehalten wird.

7. Vorrichtung nach einem der vorhergehenden Ansprüche, bei der Dampf-Einspritzeinrichtungen im Bereich des Abfalleinlasses vorgesehen sind, um eine Teildesinfektion und Vorerhitzung des Klinikabfalls dadurch zu bewirken, daß der Klinikabfall direkt erhitzt wird, bevor er in der Kammer indirekt erhitzt wird.

8. Verfahren zur Wärmebehandlung von Klinikabfall, wobei das Verfahren folgende Schritte umfasst: Fördern des zu behandelnden Abfalls durch eine Desinfektionskammer mit einer im wesentlichen konstanten Geschwindigkeit, Anwenden von Hitze, um so den Abfall indirekt zu erhitzen, während er sich durch die Kammer bewegt, Überwachen der Temperatur im Inneren der Kammer und Verändern der angewendeten Hitze in Reaktion auf die überwachte Temperatur, um ein gewünschtes stationäres Temperaturprofil im Inneren der Kammer und längs der Förderrichtung des Materials aufrechtzuerhalten, wobei dieses Temperaturprofil über einer gewünschten minimalen Desinfektionstemperatur liegt und eine Maximaltemperatur in der Nähe des Einlasses aufweist.

## Revendications

1. Appareil pour le traitement thermique de déchets hospitaliers, l'appareil comprenant une chambre de désinfection fermée (12), comprenant un orifice d'entrée des déchets (14), et un orifice de sortie des déchets (18), une vanne (30) située au niveau de l'orifice d'entrée afin de fournir un joint étanche aux gaz tout en permettant aux déchets de passer dans la chambre, des moyens de transport (16) pour transporter les déchets dans une direction de transport de l'orifice d'entrée vers l'orifice de sortie, les moyens de transport étant entraînés à une vitesse constante souhaitée, des moyens chauffants (17, 24) pour chauffer les moyens de transport et/ou le logement de la chambre afin de chauffer l'intérieur de la chambre pour un chauffage indirect des déchets alors qu'ils sont transportés entre l'orifice d'entrée et l'orifice de sortie, des moyens de détection de la température pour détecter la température de l'intérieur de la chambre et des moyens de commande informatique (50) reliés de manière opérationnelle aux moyens de détection de la température et aux moyens chauffants, les moyens de commande informatique contrôlant les moyens chauffants afin de maintenir un profil de température d'état stable souhaité tout au long de la direction de transport à l'intérieur de la chambre entre lesdits orifices d'entrée et de sortie, le profil de température le long d'une longueur souhaitée de l'intérieur de la chambre étant au-dessus d'une température de désinfection minimale souhaitée et présentant une température maximale de manière adjacente audit orifice d'entrée.

2. Appareil selon la revendication 1 comprenant en outre des moyens de fourniture d'eau (82, 92) pour fournir de l'eau sur les déchets dans la zone de l'orifice d'entrée, et des moyens de détection du degré hygrométrique pour détecter le degré hygrométrique à l'intérieur de la chambre, les moyens de fourniture d'eau et les moyens de détection du degré hygrométrique étant reliés de manière opérationnelle auxdits moyens de commande informatique (50), les moyens de commande informatique étant agencés pour commander la quantité d'eau fournie par les moyens de fourniture d'eau afin de maintenir le degré hygrométrique à l'intérieur de la chambre au-dessus d'une valeur minimale prédéterminée.

3. Appareil selon la revendication 1 ou 2, comprenant en outre des moyens d'évacuation des gaz communiquant avec l'intérieur de la chambre, un aspirateur communiquant avec les moyens d'évacuation via des moyens de filtre, l'aspirateur étant capable de retirer les gaz de la chambre à divers débits d'écoulement, et des moyens de détection de pression pour détecter la pression à l'intérieur de la chambre, les moyens de commande informatique étant reliés de manière opérationnelle à l'aspirateur et aux moyens de détection de pression et étant agencés pour faire varier l'écoulement de gaz retirés par l'aspirateur afin de maintenir une pression souhaitée dans la chambre.

4. Appareil selon la revendication 3 dans lequel les moyens de filtre communiquent avec les moyens d'évacuation via des moyens de condensation, les moyens de condensation étant activables pour condenser la vapeur d'eau des gaz d'échappement avant d'atteindre les moyens de filtre.

5. Appareil selon la revendication 4 comprenant un transporteur d'alimentation fermé pour amener les déchets hospitaliers audit orifice d'entrée des déchets, le transporteur d'alimentation étant chauffé, de préférence par de l'eau condensée provenant desdits moyens de condensation, afin de préchauffer les déchets hospitaliers avant de pénétrer dans ladite chambre.

6. Appareil selon l'une quelconque des revendications précédentes comprenant en outre un transporteur-étireur situé au niveau de l'orifice de sortie de la chambre, le transporteur-étireur étant agencé pour recevoir les déchets traités provenant dudit orifice de sortie et pour transporter les déchets traités vers un emplacement d'évacuation, le transporteur-étireur étant de préférence chauffé afin de maintenir la température des déchets traités au-dessus d'une température minimale souhaitée.

7. Appareil selon l'une quelconque des revendications précédentes dans lequel des moyens d'injection de vapeur d'eau sont prévus dans la zone dudit orifice d'entrée des déchets pour réaliser une désinfection partielle et un préchauffage des déchets hospitaliers en chauffant directement les déchets hospitaliers avant qu'ils ne soient indirectement chauffés dans ladite chambre.

8. Procédé de traitement thermique de déchets hospitaliers, le procédé comprenant les étapes consistant à transporter des déchets à traiter à travers une chambre de désinfection à un débit sensiblement constant, à appliquer de la chaleur afin de chauffer indirectement les déchets à mesure qu'ils passent à travers la chambre, à contrôler la température à l'intérieur de la chambre et à faire varier la chaleur appliquée en réponse à la température programmée afin de maintenir un profil de température d'état stable souhaité à l'intérieur de la chambre et le long de la direction du transport de la matière, ledit profil de température étant au-dessus d'une température de désinfection minimale souhaitée et comprenant une température maximale de manière adjacente audit orifice d'entrée.
